(19) Europäisches Patentamt
European Patent Office
Office européen des brevets

(11) EP 0 147 804 B2

(12) NEW EUROPEAN PATENT SPECIFICATION

(45) Date of publication and mention of the opposition decision:
**03.01.2001 Bulletin 2001/01**

(45) Mention of the grant of the patent:
**26.09.1990 Bulletin 1990/39**

(21) Application number: **84115960.1**

(22) Date of filing: **20.12.1984**

(51) Int Cl.[7]: **B01J 20/22**, B01D 15/08, B01J 20/32

(54) **Method of separating optical isomers and geometrical isomers**

Verfahren zur Trennung optischer und geometrischer Isomere

Procédé de séparation de isomères optiques et de isomères géometriques

(84) Designated Contracting States:
**BE CH DE FR GB LI NL**

(30) Priority: **28.12.1983 JP 24566783**
**05.04.1984 JP 6808884**
**05.04.1984 JP 6808984**
**11.04.1984 JP 7248384**

(43) Date of publication of application:
**10.07.1985 Bulletin 1985/28**

(73) Proprietor: **DAICEL CHEMICAL INDUSTRIES, LTD.**
**Sakai-shi Osaka-fu 590 (JP)**

(72) Inventors:
- **Okamoto, Yoshio**
  **Amagasaki-shi Hyogo (JP)**
- **Hatada, Koichi**
  **Osaka (JP)**
- **Shibata, Tohru**
  **Himeji-shi Hyogo (JP)**
- **Okamoto, Ichiro**
  **Himeji-shi Hyogo (JP)**
- **Nakamura, Hiroyuki**
  **Himeji-shi Hyogo (JP)**
- **Namikoshi, Hajime**
  **Himeji-shi Hyobe (JP)**
- **Yuki, Yoichi**
  **Himeji-shi Hyogo (JP)**

(74) Representative: **Grünecker, Kinkeldey, Stockmair & Schwanhäusser Anwaltssozietät**
**Maximilianstrasse 58**
**80538 München (DE)**

(56) References cited:

**EP-A- 0 064 833** **EP-A- 0 316 270**
**EP-A- 0 527 236** **EP-B- 0 025 639**
**EP-B- 0 047 064** **DE-B- 1 013 655**
**FR-A- 2 422 699** **GB-A- 2 092 470**
**JP-A- 537 759** **US-A- 2 032 748**
**US-A- 2 181 906** **US-A- 4 318 819**
**US-A- 4 322 310** **US-A- 4 324 681**
**US-A- 5 066 793**

- **CHEMICAL ABSTRACTS, vol. 101, 3rd September 1984, page 679, abstract no. 83260n, Columbus, Ohio, US; Y. OKAMOTO et al.: "Chromatographic resolution. 6. Useful chiral packing materials for high-performance liquid chromatographic resolution. Cellulose triacetate and tribenzoate coated on macroporous silica gel", & CHEM. LETT. 1984, (5), 739-42**
- **CHEMICAL ABSTRACTS, vol. 100, 1984, page 108, abstract no. 105399y, Columbus, Ohio, US & CS-A-205 718**
- **Hesse, Hagel Liebigs Anm. Chem. 1976, 996 - 1008**
- **Chromatographische Racemattrennungen an Cellulosetriacetat, Dissertationsschrift vorgelegt von R. Hagel, Erlangen 1976**
- **Römpps Chemie-Lexikon, 9. Auflage Bd. 1 1989, Seiten 613 und 616**
- **Hesse, Hagel Chromatographia, Vol. 6, No. 6, June 1973, Seiten 277 bis 280**
- **Firmenprospekt "SEPHADEX" der Fa. Pharmacia, 1971**
- **Pirkle, W.H. et al. (1980) J. Chromat. 192, 143-158**
- **J.Am.Chem.Soc. (1981) 103, 6971-6973**
- **Introduction to Modern Liquid Chromatography, 2.Ed. 1979, p.34-36**



Printed by Jouve, 75001 PARIS (FR)

**(Cont. next page)**

Remarks:

The file contains technical information submitted after the application was filed and not included in this specification

## Description

The invention relates to methods of separating optical isomers and geometrical isomers from a mixture.

Generally, the physiological activity of a racemic compound often differs from that of an optically active compound. For example, in the fields of medicines, pesticides or the like, it is sometimes necessary to resolve optical isomers for the purposes of preventing adverse reactions and improving the medicinal effects per unit dose. Mixtures of optical isomers have been separated by crystallization processes or diastereomer processes. However, the compounds which can be optically resolved by these processes are limited and most of these processes require a long time. Under these circumstances, development of a convenient chromatographic resolution process has eagerly been demanded.

The chromatographic resolution of optical isomers has been investigated for a long time. For instance, cellulose and triacetate derivatives thereof have been successfully used as column-chromatographic resolving agents in optical resolutions. The cellulose and cellulose triacetate are those belonging to cellulose I and cellulose triacetate I, respectively. However, substances which can be resolved by said cellulose or a derivative thereof are limited and the resolving ability of them is insufficient.

EP-A-0064833 discloses a HPLAC-process for separation of at least one biological or related substance from a mixture by means of a binding material comprising a ligand which is coupled to the matrix. The binding material is a triazinyl derivative of anthraquinones, phthalocyanines or aromatic azo compounds.

Chemical Abstracts, vol. 100, 1984, p. 108, No. 105388 y, discloses the preparation of p-aminobenzyl cellulose for immobilization of enzymes.

Liebigs Ann.Chem. 1976, p.996-1008 discloses the chromatographic resolution of racemates by means of triacetyl cellulose. It is generally disclosed on page 998 that unpolar molecules without functional groups are specifically retained on cellulose as well as on its esters, and on page 1000 that is has been found that cellulose phenylurethane and tribenzoylcellulose show in most cases reverse stereoselectivity compared to triacetyl cellulose.

The Thesis of R. Hagel, 1976, p. 134-137 discloses the preparation of cellulose triphenylcarbamate which is used for optical separation of racemates.

It is the object of the present invention to provide an improved method of separating optical isomers and geometrical isomers.

Said object is achieved by a method of separating optical isomers and geometrical isomers from a mixture by treating said mixture with a cellulose derivative having a number-average degree of polymerization of from 5 to 5000 supported on a carrier wherein at least a part of the hydrogen atoms in the hydroxy groups has been replaced by an aromatic group or a group containing an aromatic group, wherein said group may be attached to said cellulose derivative by means of an intermediate ester, ether or urethane linkage.

The object of the present invention is also achieved by a method of separating optical isomers and geometrical isomers from a mixture by treating said mixture with a cellulose derivative having a number-average degree of polymerization of from 5 to 5000 wherein at least a part of the hydrogen atoms in the hydroxy groups has been replaced by a substituted aromatic group or a group containing a substituted aromatic group, wherein said group may be attached to said cellulose denvative by means of an intermediate ester, ether or urethane linkage,as well as a method of separating optical isomers and geometrical isomers from a mixture by treating said mixture with a cellulose derivative having a number-average degree of polymerization of from 5 to 5000 wherein at least a part of the hydrogen atoms in the hydroxy groups has been replaced by an aromatic group or a group containing an aromatic group, wherein said group may be attached to said cellulose derivative by means of an intermediate ether linkage.

Though the reasons why the cellulose derivatives having an aromatic ring used in the present invention nave excellent effects for resolving optical isomers have not been elucidated yet, it be may considered that the ordered asymmetric structure of cellulose and the aromaticity and rigidity of the aromatic group exert a great influence on the resolution of optical isomers.

The cellulose derivatives used in the method of the invention have a number-average degree of polymerization of 5 to 5000, preferably 10 to 1000, and particularly 10 to 500.

The average degree of substitution of the cellulose derivatives having an aromatic ring is defined by the following formula:

$$\text{Average degree of substitution} = \frac{\text{Number of substituents in the molecule}}{\text{Number-average degree of polymerization}}$$

The average degree of substitution of the cellulose derivatives having an aromatic ring used in the method of the present invention is 1 to 3.4, preferably 1.8 to 3.2.

The unreacted hydroxyl groups in the aromatic cellulose derivatives containing aromatic rings may further be esterified, carbamoylated or etherified as far as their capacity of resolving optical isomers is maintained.

The term "aromatic group" includes those derived from an aromatic ring having 6 to 20 carbon atoms, an aralkyl group having 6 to 20 carbon atoms in the aryl portion and 1 to 4 carbon atoms in the alkyl portion and a heteroaromatic ring having 3 to 20 caroon atoms. The ring may further have a substituent thereon, such as an alkyl group, a nitro group, a halogen, an amino group, an alkyl-substituted amino group, a cyano group, a hydroxyl group and a carboxyl group.

The processes for the production of the substances used in the method of the present invention are now described. The cellulose derivatives substituted through an ester group include, for example, cellulose benzoate. The esterification reaction may be carried out by a known process ("Dai-Yuki Kagaku" 19, "Tennen Kobunshi Kagaku, I published by Asakura Book Store, p. 124). Examples of the esterifying agents include benzoyl derivatives having the following structures such as benzoyl chloride:

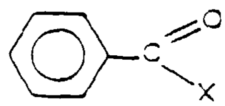

(X: leaving group)

The reaction solvent may be any solvent such as pyridine and quinoline, as far as it does not inhibit the esterification reaction. Frequently a catalyst such as 4-(N,N-dimethylamino)pyridine is effective to accelerate the reaction. Other aromatic derivatives may be obtained by the esterification reaction in the same manner as described above.

The cellulose derivatives substituted through an ether group may be obtained by a known process for etherifying cellulose. Generally, they are obtained by reacting cellulose with an aromatic derivative having a leaving group in the presence of a base. This process has been disclosed in, for example, N. M. Bikales, L. Segel, "Cellulose and Cellulose Derivatives" p. 807 and "Dai-Yuki Kagaku" 19 published by Asakura Book Store, p. 93. Processes for producing cellulose ethers having an aromatic ring of a high degree of substitution include that of Huseman et al. ("Makromol. Chem.", *176,* 3269 (1975)) and that of Nakano et al. ("The Proceedings of ISWPC" #1983, Vol. 1, 33).

The cellulose derivatives substituted through a urethane group may be produced by a conventional process wherein an isocyanate is reacted with an alcohol to form a urethane.

For example, these compounds may be produced by reacting an isocyanate having an aromatic ring with cellulose in the presence of a Lewis base catalyst such as a tertiary amine base or a Lewis acid catalyst such as a tin compound.

The disubstituted urethanes may be synthesized in the same manner as in the above-mentioned esterification reaction using a disubstituted carbamoyl halide or the like.

The resolving agents used in the method of the present invention are preferably employed in a chromatographic method. Preferred chromatographic methods include liquid, thin layer and gas chromatography.

When they are used in liquid or gas chromatography, they are packed into a column directly or in a form supported on a carrier or a capillary column is coated with said cellulose derivative.

Since the cellulose derivative is preferably in the form of granules, it is preferably ground or shaped into beads. The particle size which varies depending on the size of a column or plate used is generally 1 µm to 10 µm, preferably 1 to 300 µm. The particles are preferably porous.

It is preferred to support the aromatic ring-containing cellulose derivative on a carrier to improve the resistance thereof to pressure, to prevent swelling or shrinkage thereof due to solvent exchange or to reduce the number of theoretical plates. The suitable size of the carrier which varies depending on the size of the column or plate used is generally 1 µm to 10mm, preferably 1 to 300 µm. The carrier is preferably porous and has an average pore diameter of $10^{-7}$ cm (10 Å) to 100 µm, preferably $5 \times 10^{-7}$ to $5 \times 10^{-4}$ cm (50 to 50,000 Å). The amount of said cellulose derivative to be supported is 1 to 100 wt.%, preferably 5 to 50 wt.%, based on the carrier. The carrier preferably has a ratio of the pore size to the particle size in the range of not larger than 0.1.

The aromatic ring-containing cellulose derivative may be supported on the carrier by either chemical or physical means. For example, the cellulose derivative is dissolved in a suitable solvent, then the solution is mixed uniformly with a carrier and the solvent is distilled off under reduced pressure or by heating. Alternatively, the cellulose derivative is dissolved in a suitable solvent, the resulting solution is mixed homogeneously with a carrier and the mixture is dispersed in a liquid incompatible with said solvent by stirring to diffuse the solvent. The cellulose derivative thus supported on the carrier may be crystallized, if necessary, by heat treatment or the like. Further, the state of the supported cellulose derivative and accordingly its resolving power can be modified by adding a small amount of a solvent to temporarily swell or dissolve it and then distilling the solvent off.

Both porous organic and inorganic carriers may be used, though the latter are preferred. Suitable porous organic carriers are those comprising a high molecular substance such as polystyrene, polyacrylamide or polyacrylate. Suitable porous inorganic carriers are synthetic or natural products such as silica, alumina, magnesia, titanium oxide, glass, silicate or kaolin. They may be treated on the surface to improve the affinity with the resolving agent used in the method of the invention. The surface-treatment may be conducted with use of an organosilan compound or by plasma polymerization.

In using the cellulose derivatives in optical resolution, the resolving characteristics thereof may vary sometimes depending on the physical states thereof such as molecular weight, crystallinity and orientation, even though they are chemically similar. Therefore, the cellulose derivatives may be subjected to a physical or chemical treatment such as a heat treatment or etching in the course of or after imparting them a shape suitable for use.

As to the developers for liquid chromatography, solvents in which the aromatic ring-containing cellulose derivative is soluble cannot be used. However, the developers are not particularly limited when the aromatic ring-containing cellulose derivative is chemically bound to the carrier or when it is crosslinked.

In thin layer chromatography, a layer having a thickness of 0.1 to 100 mm and comprising the resolving agent in the form of particles of about 0.1 µm to 0.1 mm and a small amount of binder is formed on a supporting plate.

The aromatic ring-containing cellulose derivative may be spun into a hollow fiber in which an eluent containing the compound to be resolved flows so that the resolution is effected by virtue of the adsorption of the compound on the inner wall of the filament. In another embodiment, the cellulose derivative is spun into an ordinary filament, which is then bundled in parallel and placed in a column to take advantage of the adsorption on the surface thereof. In the membrane resolution process, the resolving agent may be used in the form of a hollow fiber or film.

The resolving agent used in the method of the present invention comprising the aromatic ring-containing cellulose derivative as a principal constituent is effective for the resolution of compounds. It is particularly effective for the resolution of optical isomers which are quite difficult to resolve in the prior art. The optical isomer mixture to be treated in the method of the invention includes a compound having an asymmetric center and one having a molecular asymmetry such that either one of the optical isomers may be preferably adsorbed on the cellulose derivative.

The term, "heteroaromatic derivative" used in the invention, preferably includes an ester between a cellulose and a carboxylic or carbamic acid having a heteroaromatic group or a substituted heteroaromatic group. The heteroaromatic group has 3 to 20 carbon atoms. Among them, an acyl group, a carbamoyl group and alkyl group are more preferable. Especially an acyl and carbamoyl are best. Embodiments are illustrated below.

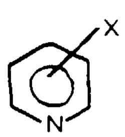 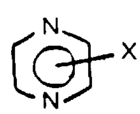 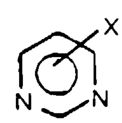 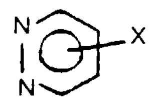

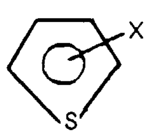 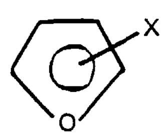 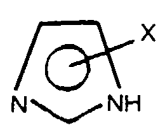 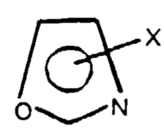

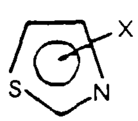 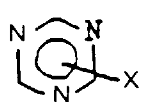 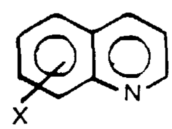 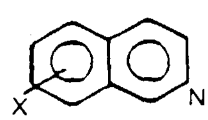

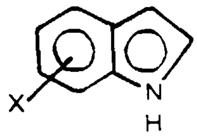 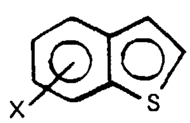 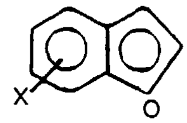

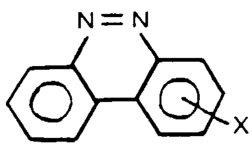 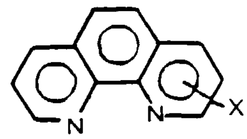

wherein X represents

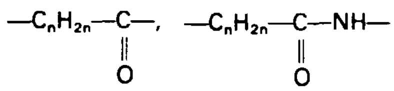

or —$C_nH_{2n}$—, n being an integer of 0 to 5, preferably 0 or 1.

The term "substituted aromatic ester" herein involves a carboxylic acid ester having an aromatic group wherein one or more hydrogen atom(s) is (are) replaced by one or more atom(s) or atomic group(s). The alcoholic moiety of the ester comprises the above-mentioned polysaccharide. The carboxylic acids preferably have acyl groups of the following formula:

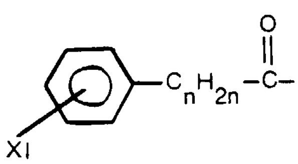 , 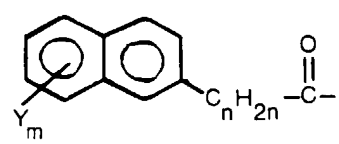 ,

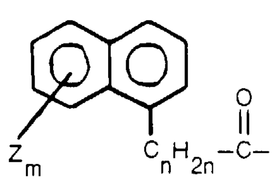

wherein X, Y and Z each represents an alkyl group, alkenyl group, alkynyl group, nitro group, halogen atom, amino group, alkyl-substituted amino group, cyano group, hydroxyl group, alkoxy group, acyl group, thiol group, sulfonyl group, carboxyl group or alkoxycarbonyl group, / and m represent the numbers of X, Y and Z groups (/ being an integer of 1 to 5, *m* being an integer of 1 to 7 and *n* being an integer of 0 to 5, preferably 0).

30 to 100%, preferably 85 to 100%, on average of the hydroxyl groups of the polysaccharide moiety in the aromatic ester should be esterified with the carboxylic acid.

The balance of the hydroxyl groups may be present in the form of free hydroxyl groups or they may be esterified, etherified or carbamoylated as far as the resolving capacity of the resolving agent is maintained.

Now, the process for the esterification to form a cellulose derivative to be used in the present invention is

described. A substituted benzoic ester may be produced by a known process (for example, "Dai-Yuki Kagaku" 19, 'Tennen Kobunshi Kagaku I' published by Asakura Book Store, p. 124). Examples of the esterifying agents include benzoyl derivatives of the following formula, particularly, benzoyl chloride:

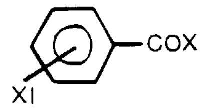

(X: leaving group)

Any solvent may be used in the reaction as far as it does not inhibit the esterification. Pyridine and quinoline are preferred. Frequently, a catalyst such as 4-(N,N-dimethylamino)pyridine is effective in accelerating the reaction.
The esters may also be obtained by reacting the corresponding carboxylic anhydride or a combination of a carboxylic acid with a suitable dehydrating agent with the polysaccharide.
Other ester derivatives used in the method of the present invention may be synthesized according to the above-mentioned process for the synthesis of the substituted benzoic esters.
The resolving agent containing a substituted aromatic ester as effective component is effective for the resolution of various compounds. Particularly, it is effective for the resolution of optical isomers which are quite difficult to resolve. Either one of the optical isomers to be resolved is selectively adsorbed on the resolving agent.
Particularly, according to the present invention, the resolving and adsorbing properties of a resolving agent are varied by introducing a substituent therein to improve the intended resolving effects, particularly, optical resolving effects. For example, the effects of cellulose tribenzoate will be compared with those of a chlorine-substituted derivative thereof. A Tröger's base which cannot be optically resolved by tribenzoate can be resolved by tris-4-chlorobenzoate with an $\alpha$-value of 1.25. Benzoin which is resolved by tribenzoate with an $\alpha$-value of 1.14 exhibits an $\alpha$-value of 1.26 in the resolution by tris-3-chlorobenzoate. Similarly, benzoin, 2-phenylcyclohexanone and mandelamide are optically resolved with tris-3,5-dichlorobenzoate far easier than with tribenzoate. Though the relationship between the remarkable change in resolving characteristics and the substitution has not fully been elucidated, the change might be caused by a complicated combination of factors such as influence of a substituent on the shape of the molecule, physicochemical properties, such as polarizability, hydrogen bonding ability and polarity, of the substituent and electronic effects of the substituent on the $\pi$-electron system of the aromatic ring.
It is thus apparent from the present invention that the substituent has a quite effective influence on the modification of the resolving characteristics of the resolving agent and the development of resolving agents having various characteristics has been made possible. These effects are expected also in various other resolving agents having polysaccharides other than cellulose as the skeleton.
Particularly, by modifying the polysaccharides with a substituent having a sufficient length, the asymmetrical structure of the polysaccharide is further developed to obtain a higher capacity of resolving the optical isomers.
The above-mentioned effects can be attained with a resolving agent containing an aralkylcarboxylic ester of a polysaccharide as effective component.
The resolving agent used in the method of the present invention exhibits preferably different adsorbing capacities on respective optical isomers of a compound.
The term "aralkylcarboxylic acids" herein involves substitution derivatives of acetic acid and they include aliphatic carboxylic acids having an aromatic substituent in the molecule and various substitution derivatives of them, preferably arylacetic acid and aryloxyacetic acid derivatives. Acrylic acid derivatives, benzoic acid derivatives and propiolic acid derivatives must be excluded even if they contain an aromatic ring. The aromatic rings include, for example, phenyl, naphthyl, phenanthryl and anthryl rings. They may be bonded with the skeleton in any manner.
Examples of these compounds include the following compounds:

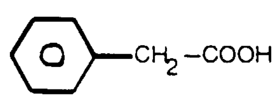

phenylacetic acid

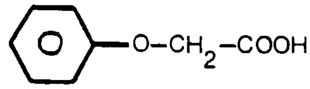

phenoxyacetic acid

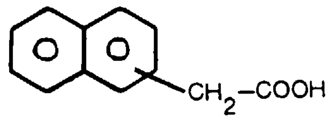

naphthaleneacetic acid (both α- and β-isomers)

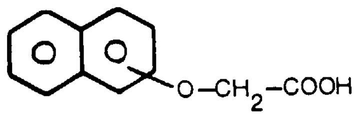

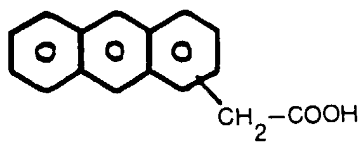

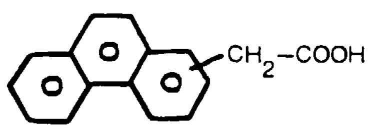

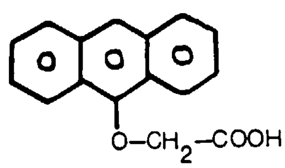

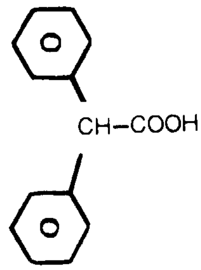

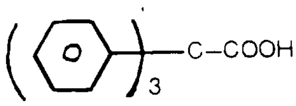

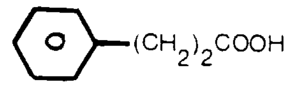

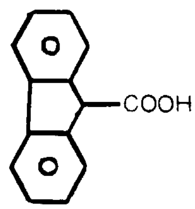

The aromatic ring may have various substituents as far as the effects of the present invention are achieved.

30 to 100%, preferably 85 to 100%, on average of the hydroxyl groups of the polysaccharides forming the polysaccharide/aralkylcarboxylic ester should be esterified with a carboxylic acid. The balance of the hydroxyl groups may be present in the form of free hydroxyl groups or they may be esterified, etherified or carbamoylated as far as the resolving capacity of the resolving agent is achieved.

The esterification for forming the compounds used in the method of the present invention may be conducted by a known process for the esterification of cellulose or amylose (for example, "Dai-Yuki Kagaku" 19, 'Tennen Kobunshi Kagaku I' published by Asakura Book Store, p. 124, reference 1). Common esterifying agents are anhydrides and halides of the corresponding carboxylic acids, particularly acid chlorides.

It is preferred to use a tertiary amine base or a Lewis acid as catalyst. Any reaction solvent may be used as far as it does not inhibit the reaction. For example, pyridine or quinoline which also acts as base is used frequently. Further, a catalyst such as 4-(N,N-dimethyl-amino)pyridine is effective in accelerating the reaction.

The corresponding carboxylic acid in combination with a suitable dehyrating agent may be reacted with the polysaccharide.

Since most of the polysaccharides used as the starting material have a low reactivity, it is preferred that they are activated by dissolution/reprecipitation or dissolution/freeze-drying treatment or by using a reaction solvent in which the polysaccharides are soluble.

Brief Description of the Drawings

Figs. 1 and 2 are charts showing the results of optical resolution effected by using packings for the optical resolution.

The invention will be illustrated below with reference to synthesis examples, examples and application examples. The particular terms used are defined below.

$$\text{volume ratio } (k') = \frac{[(\text{retention time of antipode}) - (\text{dead time})]}{(\text{dead time})}$$

$$\text{separation factor } (\alpha) = \frac{\text{(volume ratio of antipode adsorbed more strongly)}}{\text{(volume ratio of antipode adsorbed less strongly)}}$$

$$\text{rate of separation (Rs)} = \frac{2 \times \text{(distance between a peak of more strongly adsorbed antipode and that of less strongly adsorbed antipode)}}{\text{(total band width of both peaks)}}$$

Synthesis Example 1 (synthesis of cellulose benzoate)

①Synthesis of low-molecular weight cellulose triacetate

100 g of cellulose triacetate having a number-average degree of polymerization of 110 and a degree of acetylation of 2.94 was dissolved in 100 ml of acetic acid. 5.2 ml of water and 5 ml of concentrated sulfuric acid were added to the solution and the mixture was maintained at 80°C for 3 h to effect the reaction for obtaining a product of a low molecular weight. After completion of the reaction, the reaction mixture was cooled and sulfuric acid was neutralized with excess aqueous magnesium acetate solution. The solution was added to 3 l of water to precipitate cellulose triacetate of a low molecular weight. The precipitate was filtered through a G3 glass filter, dispersed in 1 l of water, further filtered and dried under vacuum. The obtained product was dissolved in methylene chloride and reprecipitated from 2-propanol. The dissolution and reprecipitation were repeated twice to purify the product, which was then dried.

From the IR and NMR spectra of the product, it was identified as cellulose triacetate. The number-average molecular weight of the product was 7900 (the degree of polymerization: 27) according to vapor pressure osmometry (CORONA 117; chloroform/1% ethanol).

②Synthesis of cellulose having a low molecular weight

5.0 g of the low-molecular weight cellulose triacetate prepared as above was dissolved in 50 ml of pyridine. 4.0 ml of 100% hydrazine hydrate was added to the solution. The mixture was left to stand at room temperature for 1 h and then heated to 90 to 100°C. The precipitate thus formed was filtered through a glass filter and washed with pyridine. The product containing pyridine was used in the subsequent reaction.

③Synthesis of low-molecular weight cellulose tribenzoate

The low-molecular weight cellulose obtained as above was dispersed in a mixture of 50 ml of pyridine and 21 ml of triethylamine. 200 mg of 4-(dimethylamino)pyridine as catalyst was added to the dispersion. 11.6 ml of benzoyl chloride was slowly added dropwise to the mixture under stirring. The resulting mixture was left to stand at room temperature for 3 h and then kept at 120°C for 10 h to complete the reaction. The resulting pyridine solution was added to a large excess of methanol. The precipitate thus formed was filtered and washed with methanol. The product was dissolved in methylene chloride and reprecipitated from ethanol. This purification process was repeated three times.

From the IR and NMR spectra of the product, it was identified as cellulose tribenzoate. From the fact that no occurrence of acylation was recognized in the NMR spectra after treating the product with acetic anhydride in pyridine, it may be concluded that the hydroxyl groups have been esterified into benzoate groups without leaving any free hydroxyl groups intact.

Synthesis Example 2 (synthesis of packing for use in optical resolution)

①Treatment of silica gel with silane

10 g of silica beads (Lichrospher SI 1000: a product of Merck & Co.) was placed in a 200 ml round-bottom flask with a side arm. After vacuum-drying in an oil bath at 120°C for 3 h, the pressure was returned to atmospheric pressure, the temperature was lowered to room temperature and $N_2$ was introduced. 100 ml of toluene which had been preliminarily distilled was added to the dry silica beads. 3 ml of diphenyldimethoxysilane (KBM 202; a product of Shin'etsu Kagaku Co., Ltd.) was added to the mixture and they were stirred together and then reacted at 120°C for 1 h. After distilling off 3 to 5 ml of toluene, the reaction was carried out at 120°C for 2 h. The mixture was filtered under suction through a glass filter (G-4), washed with 50 ml of toluene three times and then with 50 ml of methanol three times and

dried in vacuum at 40°C for 1 h.

About 10 g of the silica beads treated as above were placed in the 200 ml round-bottom flask with a side arm. After vacuum drying at 100°C for 3 h, the pressure was returned to atmospheric pressure and the mixture was cooled to room temperature. Then $N_2$ was introduced.

100 ml of distilled toluene was added to the dried silica beads. 1 ml of N,O-bis(trimethylsilyl)acetamide (a trimethylsilylating agent) was added thereto and the mixture was stirred to effect the reaction at 115°C for 3 h.

The reaction mixture was filtered through a G-4 glass filter, washed with toluene and dried under vacuum for about 4 h.

②Coating

1.6 g of the cellulose benzoate obtained in Synthesis Example 1 was dissolved in 10.0 ml of methylene chloride and the solution was filtered through a G-3 glass filter.

3.5 g of the silica gel treated with silane was mixed with 7.5 ml of said cellulose benzoate solution and the solvent was distilled off under reduced pressure.

Example 1

The silica gel packing coated with cellulose benzoate obtained in Synthesis Example 2 was packed in a stainless steel column having an inner diameter of 0.46 cm and a length of 25 cm by a slurry method (solvent: methanol). Various racemic compounds were optically resolved according to high-performance liquid chromatography using this column to obtain the results shown in Figs. 1 and 2 and Table 1.

The symbols (+ and -) in the figures and the table refer to the signs of optical rotation at 365 nm.

Fig. 1 is an optical resolution chart of trans-stilbene oxide

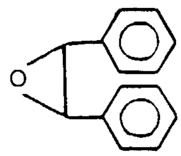

[flow rate: 0.2 ml/min) solvent: hexane/2-propanol (9:1)]. Fig. 2 is an optical resolution chart of a cyclobutane derivative:

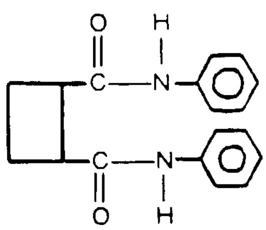

[flow rate: 0.2 ml/min, solvent: ethanol].

TABLE 1 Optical resolution of racemic compounds

| Racemic compounds | Volume ratio $k'_1$ | $k'_2$ | Separation factor α | Rate of separation (Rs) |
|---|---|---|---|---|
| trans-stilbene oxide 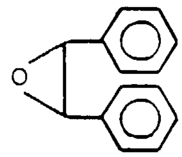 | 0.71 (+) | 1.00 (−) | 1.4 | 2.2 |
| 2-phenylcyclohexane 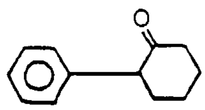 | 0.99 (−) | 1.14 (+) | 1.2 | 0.98 |
| cyclobutane derivative 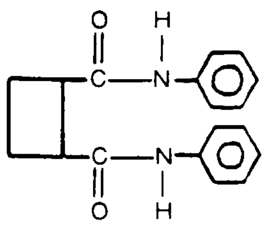 | 0.42 (+) | 0.64 (−) | 1.5 | 0.97 |
| benzoin 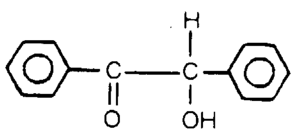 | 0.53 (+) | 0.58 (−) | 1.1 | — |

Notes:
Column: 25 cm × 0.46 cmφ
Flow rate: 0.2 ml/min, solvent: ethanol.

Comparative Example 1

140 g of cellulose triacetate produced by an ordinary homogeneous acetylation process (number-average degree of polymerization as determined by vapor pressure osmometry: 110; molecular weight distribution $\overline{Mw}/\overline{Mn}$ = 2.45, free hydroxyl group content: (0.35%) was swollen in 1.4l of acetic acid (a guaranteed reagent of Kanto Kagaku Co.). 23.2 ml of acetic anhydride, 7.0 ml of sulfuric acid and 8.4 ml of water were added thereto and the reaction was carried out at 80°C for 3 h. The reaction mixture was cooled with ice/water and sulfuric acid was neutralized with 86.8 g of 26% aqueous magnesium acetate solution. The solution thus obtained was added to a solvent mixture of water/2-propanol to precipitate cellulose acetate which was then filtered and dried. The obtained cellulose acetate was dissolved in acetone. An insoluble matter was filtered out under pressure. Water was added to the residue in such an amount that no precipitate would form. The solvent was distilled off with a rotary evaporator. The white powder thus obtained was dried under reduced pressure.

From the results of X-ray diffractometry, it was found that the resulting crystalline cellulose acetate had a crystallinity of 46% and a half width of 0.58°. The average degree of polymerization determined based on the viscosity in a solvent mixture of methanol/methylene chloride (1:1) was 23. The free hydroxyl group content of the product was 0.8%. According to electron microscope observation the product was in the form of porous particles having a diameter of 1 to 10 μm. The resolution was effected in the same manner as in Example 1 except that the triacetylcellulose was packed by a slurry process using methanol as the solvent. Trans-stilbene oxide had a resolution factor a of 1.34 and a rate of separation (Rs) of 0.91. No peak separation was observed with 2-phenylcyclohexanone and benzoin.

Synthesis Example 3

Cellulose triacetate having a number-average degree of polymerization of 110 and a degree acetylation of 2.94 was dissolved in 1 l of acetic acid. 5.2 ml of water and 5 ml of conc. sulfuric acid were added to the resulting solution and the reaction was carried out at 80°C for 3 h. The reaction mixture was cooled and sulfuric acid was neutralized with an excess amount of an aqueous magnesium acetate solution. The resulting solution was added to 3 l of water to precipitate cellulose triacetate having a reduced molecular weight. After filtration through a glass filter (G3), it was dispersed in 1 l of water. After filtration followed by vacuum drying, the obtained product was dissolved in methylene chloride and reprecipitated from 2-propanol. The dissolution and the reprecipitation was repeated twice to effect purification. The product was dried. According to the IR and NMR spectra, the product was identified as cellulose triacetate. The number-average molecular weight of the product as determined by vapor pressure osmometry was 7900, which corresponded to a number-average degree of polymerization of 27. The vapor pressure osmometry was conducted with a vapor pressure osmometer Corona 117 using a solvent mixture of chloroform/1% ethanol.

10 g of the resulting low-molecular weight cellulose triacetate was dissolved in 100 ml of pyridine. 8.0 ml of 100% hydrazine hydrate was added to the solution. The mixture was left to stand at room temperature for 1 h and then heated to 90 to 100°C. The precipitate thus formed was filtered through a glass filter and washed with pyridine. According to the IR spectrum, the resulting product was identified as cellulose.

Cellulose tribenzyl ether was synthesized from the resulting low-molecular weight cellulose by successive processes of Huseman et al. [Markromol. Chem., *176*, 3269 (1975)]. The product was identified according to NMR and IR spectra. In the IR spectrum, no absorption due to the hydroxyl group was recognized at all. This fact suggested that the degree of substitution was about 3.

NMR ($CDCl_3$): $\delta$ 7.1 (multiplets) 15H, $\delta$ 5.3～$\delta$ 2.8 (multiplets) 13H.

IR (KBr disc.): 1950 (w), 1870 (w), 1805 (w), 1740 (w), 1605 (m), 1500 (m), 740 (s), 700 (s) all due to the substituted benzene ring, 1050～1100 (vs) due to the glycoside bond.

Synthesis Example 4

10 g of silica beads (LiChrospher SI 1000; a product of Merck & Co.) was placed in a 200 ml round-bottom flask with a side arm. After vacuum-drying in an oil bath at 120°C for 3 h, $N_2$ was introduced therein. 100 ml of toluene which had been preliminarily distilled in the presence of $CaH_2$ was added to the silica beads. 3 ml of diphenyldimethoxysilane (KBM 202; a product of Shin'estu Kagaku Co., Ltd.) was added to the mixture and they were stirred together and then reacted at 120°C for 1 h. After distilling off 3 to 5 ml of toluene, the reaction was carried out at 120°C for 2 h. The mixture was filtered through a glass filter, washed with 50 ml of toluene three times and then with 50 ml of methanol three times and dried in vacuum at 40°C for 1 h.

About 10 g of the silica beads were placed in the 200 ml round-bottom flask with a side arm. After vacuum drying at 100°C for 3 h, the pressure was returned to atmospheric pressure and the mixture was cooled to room temperature. Then, $N_2$ was introduced, 100 ml of distilled toluene was added to the dried silica beads. 1 ml of N,O-bis (trimethylsilyl)acetamide (a trimethylsilylating agent) was added thereto and the mixture was stirred to effect the reaction at 115°C for 3 h. The reaction mixture was filtered through a glass filter, washed with toluene and dried under vacuum for about 4 h.

Tribenzylcellulose obtained in Synthesis Example 3 was dissolved in chloroform. Methanol in an amount four times as much as chloroform was added to the solution to divide it into a soluble part and an insoluble part. 1.2 g of the soluble part was dissolved in a solvent mixture of methylene chloride and benzene (5 ml: 2.5 ml). 6 ml of the resulting solution was mixed with 3.2 g of silane-treated silica gel. The solvent was distilled off under reduced pressure. The resulting silica beads were used as a packing for use in optical resolution.

Example 2

The silica beads carrying tribenzylcellulose obtained in Synthesis Example 4 were packed in a stainless steel column having a length of 25 cm and an inner diameter of 0.46 cm by slurry process. The high performance liquid chromatograph used was TRIROTAR-SR (a product of Nihon Bunko Kogyo Co., Ltd.) and the detector used was UVIDEC-V. The flow rate was 0.2 ml/min and ethanol was used as the solvent. The results of resolution of various racemic compounds are shown in Table 2.

TABLE 2 Optical resolution of racemic modifications

| Racemic compound | Volume ratio k'$_1$ | k'$_2$ | Separation factor α | Rate of separation (Rs) |
|---|---|---|---|---|
| 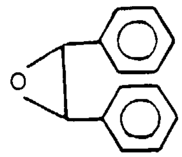 | 0.75 | 0.99 | 1.32 | 1.50 |
| 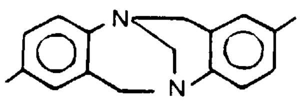 | 0.66 | 0.99 | 1.46 | 1.12 |

Synthetic Example 5

1 g of cellulose (cellulose for column chromatography; a product of Merck & Co.) was dispersed in 50 ml of dry pyridine. 8 ml of phenyl isocyanate was added to the dispersion and the mixture was stirred at 110°C. After 16 h, the reaction mixture was poured into 1 l of methanol. A white solid thus formed was filtered and dried at room temperature for 2 h and then at 60°C for 3 h under reduced pressure. Yield: 1.45 g.

The product was substantially completely soluble in chloroform, methylene chloride and dioxane. The product was identified as cellulose trisphenylcarbamate according to the IR and NMR spectra. The degree of polymerization as determined according to GPC was 200.

| *Elementary analysis* | | | |
|---|---|---|---|
| Found | C, 60.93%; | H, 4.68%; | N, 7.93% |
| Calculated for $(C_{27}H_{25}N_3O_8)_n$ | C, 62.42%; | H, 4.85%; | N, 8.09% |

Synthesis Example 6

102 g of silica gel (Lichrospher SI 4000; a product of Merck & Co.) was dried at 180°C for 2 h and then dispersed in a mixture of 600 ml of dry benzene, 6 ml of pyridine and 20 ml of 3-aminopropyltriethoxysilane to effect the reaction under reflux for 16 h. After completion of the reaction, the reaction mixture was poured into 2 l of methanol and the modified silica gel was filtered.

0.76 g of the cellulose trisphenylcarbamate obtained in Synthesis Example 5 was dissolved in a solvent mixture of 10 ml dioxane and 5 ml of ethanol. After removing a very small amount of the insoluble matter, 3.0 g of the modified silica gel was mixed with 5 ml of the solution and the solvent was distilled off under reduced pressure. This process was repeated twice to obtain cellulose trisphenylcarbamate-carrying silica gel.

Example 3

The carrying silica gel prepared in Synthesis Example 6 was packed in a stainless steel column having a length of 25 cm and an inner diameter of 0.46 cm by slurry process. The high-performance liquid chromatograph used was TRIROTAR-II (a product of Nihon Bunko Kogyo Co., Ltd.) and the detector used was UVIDEC-III and DIP-181 polarimeter. The solvents used were (1) hexane/-2-propanol (mixing volume ratio: 90:10), (2) hexane/2-propanol (80:20), (3) hexane/2-propanol/diethylamine (80:20:0.001), (4) ethanol/ water (50:50) and (5) ethanol water (70:30). The flow rate was 0.5 ml/min and the column temperature was 25°C in all the cases.

The results of the resolution of various racemic compounds are shown in Tables 3—1 to 3—4 and those of the separation of achiral compounds are shown in Table 4.

*Measurement of molecular weight*

The molecular weight was determined by a GPC method using a calibration curbe of standard polystyrene. The GPC column used was Shodex A 80 M and the solvent was tetrahydrofuran.

TABLE 3—1 Optical resolution with cellulose trisphenylcarbamate

| Racemic compound | Eluent[a] | $k'_1$ (optical rotatory power)[b] | α | Rs |
|---|---|---|---|---|
| 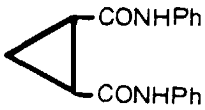 | 1 | 1.92 | 1.46 | 1.27 |
| 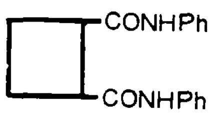 | 1 | 3.64 | 1.20 | — |
| 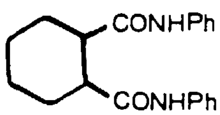 | 1 | 0.65 | 1.88 | 1.06 |
| 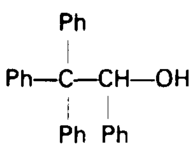 | 1 | 2.39 | 1.23 | 0.77 |
| 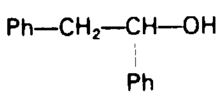 | 1 | 1.32 | 1.13 | 0.58 |
| 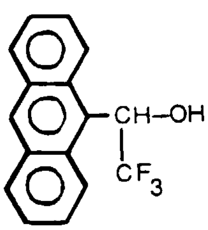 | 1 | 1.42 | 1.47 | 1.38 |

TABLE 3—2

| Racemic compound | Eluent[a)] | k'$_1$ (optical rotatory power)[b)] | α | Rs |
|---|---|---|---|---|
| 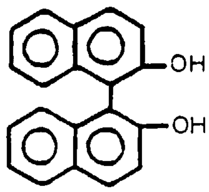 | 1 | 6.38 | 1.19 | 0.97 |
| 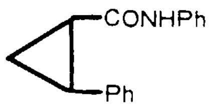 | 1 | 5.92 | 1.95 | 3.36 |
| 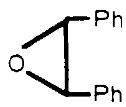 | 1 | 0.58 (+) | 1.53 | 2.00 |
| 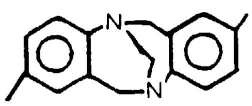 | 1 | 1.02 | 1.39 | 1.73 |
| 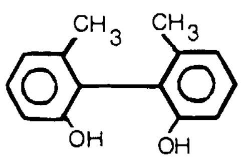 | 1 | 2.06 (−) | 1.68 | 2.56 |
| 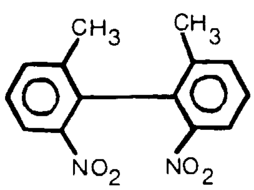 | 1 | 3.67 (−) | 1.52 | 3.50 |

TABLE 3—3

| Racemic compound | Eluent[a)] | $k'_1$ (optical rotatory power)[b)] | α | Rs |
|---|---|---|---|---|
| $Cr(acac)_3$ | 1 | 2.00 (−) | 1.48 | 1.14 |
| $Co(acac)_3$ | 1 | 2.24 (+) | 1.31 | 0.75 |
| 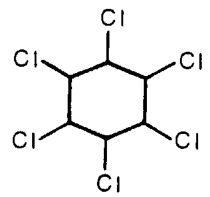 | 1 | 0.50 (+) | 3.09 | 2.26 |
| 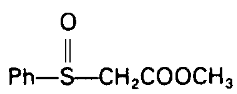 | 2 | 1.07 (+) | 1.08 | 0.53 |
| 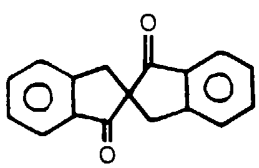 | 3 | 6.38 (−) | 1.24 | 1.07 |
| 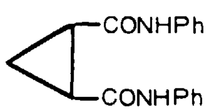 | 4 | 4.27 (−) | 1.22 | 0.86 |
| 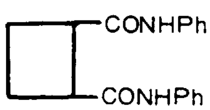 | 4 | 5.35 (+) | 1.12 | — |

TABLE 3—4

| Racemic compound | Eluent[a] | k'₁ (optical rotatory power)[b] | α | Rs |
|---|---|---|---|---|
| 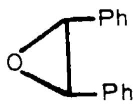 | 4 | 11.3 (+) | 1.36 | 1.74 |
| 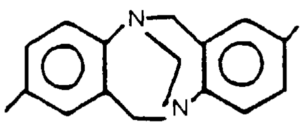 | 4 | 6.70 (+) | 1.17 | 0.85 |
| 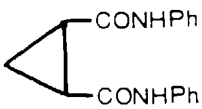 | 5 | 0.49 (−) | 1.13 | — |
| 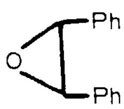 | 5 | 1.85 (+) | 1.31 | 1.58 |
| 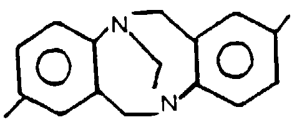 | 5 | 1.30 (+) | 1.21 | 0.9 |
| 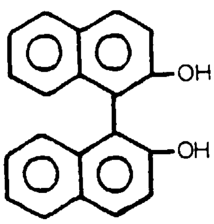 | 5 | 0.85 (+) | 1.27 | 0.68 |

a) eluent 1: hexane/2-propanol (90/10)
eluent 2: hexane/2-propanol (80/20)
eluent 3: hexane/2-propanol/diethylamine (80:20:0.001)
eluent 4: ethanol/water (50:50)
eluent 5: ethanol/water (70:30)
b) wavelength: 365 nm

TABLE 4
Separation of archiral compounds with cellulose trisphenylcarbamate[a)]

| Compound | Retention time (min) |
|---|---|
| 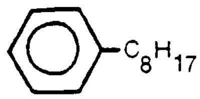 | 6.30 |
| 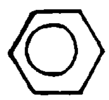 | 6.80 |
| 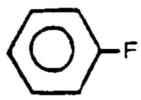 | 6.90 |
| 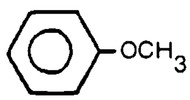 | 7.60 |
| 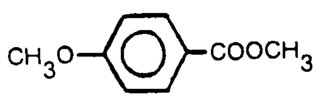 | 8.33 |
| 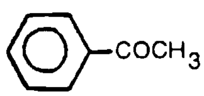 | 8.65 |
| 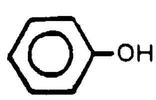 | 8.95 |
| 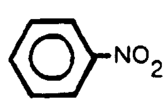 | 10.05 |

TABLE 4 (continued)

| Compound | Retention time (min) |
|---|---|
| 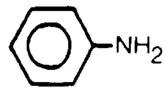 | 15.25 |
| 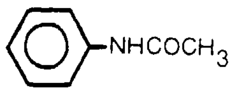 | 16.30 |
| $CH_3COOC_2H_5$ | 8.85 |
| $Ch_3COCH_3$ | 9.75 |
| $Et_3N$ | 6.75 |
| $Et_2NH$ | 7.70 |

a) eluent: hexane/2 propanol (80:20)

Synthesis Example 7

Cellulose triacetate having a number-average degree of polymerization of 110 and a degree of acetylation of 2.49 was dissolved in 1 l of acetic acid. 5.2 ml of water and 5 ml of conc. sulfuric acid were added to the resulting solution and the reaction was carried out at 80°C for 3 h. The reaction liquid was cooled and sulfuric acid was neutralized with an excess amount of an aqueous magnesium acetate solution. The resulting solution was added to 3 l of water to precipitate cellulose triacetate having a reduced molecular weight. After filtration through a glass filter (G 3), it was dispersed in 1 l of water. After filtration followed by vacuum drying, the obtained product was dissolved in methylene chloride and then reprecipitated from 2-propanol. The dissolution and the reprecipitation were repeated twice to effect the purification. The product was dried. According to the IR and NMR spectra, the product was identified as cellulose triacetate. The number-average molecular weight of the product as determined by vapor pressure osmometry was 7900 which corresponded to the number-average degree of polymerization of 27. The vapor pressure osmometry was conducted with a vapor pressure osmometer Corona 117 using a solvent mixture of chloroform/1% ethanol.

10.0 g of the resulting low-molecular weight cellulose triacetate was dissolved in 100 ml of pyridine, 8.0 ml of 100% hydrazine hydrate was added to the solution. The mixture was left to stand at room temperature for 1 h and then heated to 90 to 100°C. The precipitate thus formed was filtered through a glass filter and washed with pyridine. According to the IR spectrum, the obtained product was identified as cellulose.

100 ml of dry pyridine was added to a mixture of 5 g of the resulting low molecular weight cellulose and a small amount of pyridine to obtain a dispersion. 100 ml of benzene was added to the dispersion to remove water. After distillation through a rectifier tube, the remaining suspension of the low molecular weight cellulose in pyridine was heated to 60 to 70°C. 16.3 ml of phenyl isocyanate was added dropwise to the suspension under stirring. The mixture was kept at 100 to 105°C for 3 h 35 min. Pyridine and phenyl isocyanate were distilled off under reduced pressure and the reaction mixture was dissolved in methylene chloride. The by-product insoluble in methylene chloride was filtered

through a glass filter (G 3) and the soluble matter was fractionated with 2-propanol. The 2-propanol-insoluble product was obtained in an amount of 5.67 g as a light yellow solid. According to the IR and NMR spectra, the product was identified as cellulose trisphenylcarbamate.

IR spectrum: 3500 $cm^{-1}$ ($v_{NH}$), 3300 $cm^{-1}$ ($v_{NH}$), 1700 $cm^{-1}$ ($v_{c=0}$), 1530 $cm^{-1}$ ($v_{NH}$),

NMR spectrum: 18 H Broad singlet centered at δ7 7 H multiplets δ6.0~3.0.

Synthesis Example 8

Silica gel (Lichrospher SI 1000, a product of Merck & Co.) was dried by heating to 120 to 150°C in dry nitrogen stream for 2 to 10 h. 20 g of the dried silica gel was suspended in 100 ml of anhydrous benzene. 6 g of 3-aminopropyltrimethoxysilane was added thereto and the mixture was heated under reflux in dry nitrogen stream. While removing formed methanol from the reaction system, the reaction was carried out for 5 to 10 h. After completion of the reaction, the reaction mixture was cooled to room temperature and filtered through a glass filter. The resulting modified silica gel was washed with anhydrous benzene and dried at 40°C in vacuum.

6 g of the silica gel treated with the aminopropylsilane was dried at 80°C under reduced pressure for 2 h and then dispersed in 50 ml of dry methylene chloride. 2 ml of the triethylamine and 1 ml of phenyl isocyanate were added to the dispersion and mixed well. The mixture was left to stand for one day and then heated to 40°C for 1 h. The solvent was removed by decantation and the residue was washed with methylene chloride, ethanol and acetone and dried.

0.9 g of cellulose trisphenylcarbamate obtained in Synthesis Example 7 was dissolved in 4.5 ml of methylene chloride. The resulting solution was mixed with 3.5 g of the modified silica gel. The solvent was distilled off under reduced pressure to obtain a cellulose trisphenylcarbamate-carrying silica gel.

Example 4

The silica gel carrying cellulose trisphenylcarbamate obtained in Synthesis Example 8 was packed in a stainless steel column having a length of 25 cm and an inner diameter of 0.46 cm by a slurry method. The high-performance liquid chromatograph used was TRIROTAR-SR (a product of Nihon Bunko Kogyo Co., Ltd.) and the detector used was UVIDEC-V. The flow rate was 0.2 ml/min and hexane/2-propanol (9:1) was used as solvent. The results of various racemic compounds are shown in Table 5.

TABLE 5 Optical resolution of racemic compounds

| Racemic compound | Volume ratio k'₁ | Volume ratio k'₂ | Resolution factor α | Rate of separation Rs |
|---|---|---|---|---|
| 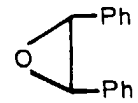 | 1.13 | 1.39 | 1.23 | 1.89 |
| 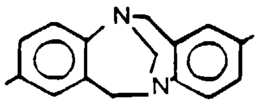 | 2.18 | 2.60 | 1.19 | 1.30 |
| 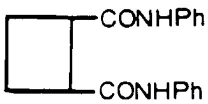 | 4.50 | 5.60 | 1.24 | 0.90 |
| 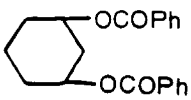 | 2.33 | 2.70 | 1.16 | 0.5 |
| 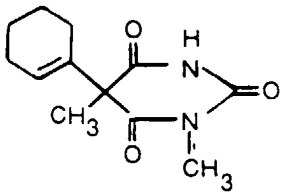 | 11.9 | 13.3 | 1.12 | — |

Synthesis Example 9

10 g of silica beads (LiChrospher SI 1000; a product of Merck & Co.) was placed in a 200 ml round-bottom flask with a side arm. After vacuum-drying in an oil bath at 120°C for 3 h, $N_2$ was introduced. 100 ml of toluene which had been preliminary distilled in the presence of $CaH_2$ was added to the silica beads. 3 ml of diphenyldimethoxysilane (KBM 202; a product of Shin'etsu Kagaku Co., Ltd) was added to the mixture and they were stirred together and then reacted at 120°C for 1 h. After distilling off 3 to 5 ml of toluene, the reaction was carried out at 120°C for 2 h. The mixture was filtered through a glass filter, washed with 50 ml of toluene three times and then with 50 ml of methanol three times and dried in vacuum at 40°C for 1 h.

About 10 g of the silica beads were placed in the 200 ml round-bottom flask with a side arm. After vacuum drying at 100°C for 3 h, the pressure was returned to atmospheric pressure and the mixture was cooled to room temperature. Then, $N_2$ was introduced, 100 ml of distilled toluene was added to the dried silica beads. 1 ml of N,O-bis (triethylsilyl)acetamide (a trimethylsilylating agent) was added thereto and the mixture was stirred to effect the reaction at 115°C for 3 h. The reaction mixture was filtered through a glass filter, washed with toluene and dried under vacuum for about 4 h.

Synthesis Example 10

Cellulose triacetate (a product of Daicel Ltd.) having a number-average degree of polymerization of 110 and a degree of substitution of 2.97 was dissolved in 1 l of acetic acid (a product of Kanto Kagaku Co.). 5.2 ml of water and 5 ml of conc. sulfuric acid were added to the resulting solution and the reaction was carried out at 80°C for 3 h. The reaction mixture was cooled and sulfuric acid was neutralized with an excess amount of an aqueous magnesium acetate solution. The resulting solution was added to 3 l of water to precipitate cellulose triacetate having a reduced

molecular weight. After filtration through a glass filter (G3), it was dispersed in 1 l of water. After filtration followed by vacuum drying, the obtained product was dissolved in methylene chloride and reprecipitated from 2-propanol. The dissolution and the reprecipitation were repeated twice to effect purification. The product was dried. According to the IR and NMR spectra, the product was identified as cellulose triacetate. The number-average molecular weight of the product as determined by vapor pressure osmometry was 7900, which corresponded to a number-average degree of polymerization of 27. The vapor pressure osmometry was conducted with a vapor pressure osmometer Corona 117 using a solvent mixture of chloroform/1% ethanol.

60 g of the obtained cellulose triacetate was dispersed in 200 ml of 2-propanol. 60 ml of 100% hydrazine hydrate (a product of Nakai Kagaku Co.) was slowly added dropwise to the dispersion under gentle stirring. The suspension was maintained at 60°C for 3 h and the resulting cellulose was filtered through a glass filter, washed with acetone repeatedly and vacuum-dried at 60°C. In the IR spectrum of the product, no absorption band due to the carbonyl group at around 1720 $cm^{-1}$ was observed and the IR spectrum coincided with that of cellulose.

Synthesis Example 11

70 ml of dehydrated pyridine, 7.7 ml of dehydrated triethylamine and 50 mg of 4-dimethylaminopyridine were added to 1.5 g of the cellulose obtained in Synthesis Eample 10. 12.2 g of thiophene-2-carbonyl chloride was added to the mixture under stirring. The mixture was stirred at 100°C for 5 h to carry out the reaction. After cooling, the product was added to 400 ml of ethanol under stirring to form precipitates, which were filtered through a glass filter and then washed thoroughly with ethanol. After vacuum drying, the product was dissolved in 30 ml of methylene chloride. The insoluble matter was removed and the product was reprecipitated from 400 ml of ethanol. The precipitate was filtered and washed with ethanol. After removing the liquid, the product was dried.

Thus, 3.9 g of cellulose thiophene-2-carboxylate was obtained.

The product was dissolved in methylene chloride and the solution was applied on a sodium chloride tablet and dried. The infrared absorption spectrum of the product had the following characteristic absorption bands:

3100 $cm^{-1}$ : stretching vibration of aromatic C—H,
1720 $cm^{-1}$ : stretching vibration of C=O of carboxylic acid ester,
1360, 1420, 1520 $cm^{-1}$ : stretching vibration of thiophene ring,
260 $cm^{-1}$ : stretching vibration of C—O of ester,
1060 to 1160 $cm^{-1}$ : stretching vibration of C—O—C of cellulose, and
860 $cm^{-1}$ : out-of-plane deformation vibration of disubstituted thiophene.

Substantially no absorption at around 3450 $cm^{-1}$ due to OH of cellulose was observed. This fact suggested that the product substantially comprised a trisubstituted compound. In the proton NMR spectrum determined in $CDCl_3$, the characteristic absorptions were as follows:

6.8 to 7.8 ppm: proton of thiophene ring,
2.8 to 5.4 ppm: protons of the cellulose ring and methylene in position 6.

The ratio of the thiophene ring proton to the cellulose proton was about 9:7, which coincided with that of the trisubstituted compound.

According to elementary analysis of the product, the sulfur content thereof was 19.40% which suggested that the product substantially comprised a trisubstituted compound.

Example 5

1.2 g of cellulose tris(thiophene-2-carboxylate) obtained in Synthesis Example 11 was dissolved in 7.5 ml of dichloromethane. The solution was filtered. The silica gel particles obtained in Synthesis Example 9 were impregnated with 7.5 ml of the resulting solution. The solvent was distilled off under reduced pressure to obtain a powdery, supported material.

Application Example 1

The silica beads carrying cellulose tris(thiophene-2-carboxylate) obtained in Example 5 were packed in a stainless steel column having a length of 25 cm and an inner diameter of 0.46 cm by a slurry process. The high-performance liquid chromatograph used was TRIROTAR-RS (a product of Nihon Bunko Kogyo Co., Ltd.) and the polarimeter detector was DIP-181 (a product of Nihon Bunko Kogyo Co., Ltd.). The results of the resolution of trans-stilbene oxide are shown in Table 6.

In the determination effected by using a polarimeter as detector of the high-performance chromatograph, the terms were defined as follows:

$$I' = \frac{\text{(time required for attaining the top of the peak of antipode with the polarimeter detector)} - \text{(dead time)}}{\text{(dead time)}}$$

$$\beta = \frac{\text{(I' of antipode adsorbed more strongly)}}{\text{(I' of antipode adsorbed less strongly}}$$

TABLE 6

| Racemic compound | $I_1'$ | $I_2'$ | β | Flow rate ml/min |
|---|---|---|---|---|
| 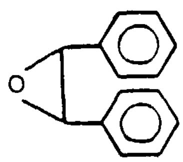 | 1.88 | 2.28 | 1.21 | 1.0 |

Solvent: hexane/2-propanol (9:1).

Synthesis Example 12

Synthesis of cellulose tris-3-chlorobenzoate

3.0 g of cellulose obtained in Synthesis Example 10 and 0.05 g of 4-dimethylaminopyridine (a product of Aldrich Chemical Company) were suspended in a liquid mixture of 50 ml of pyridine and 15 ml of triethylamine. 25 g of m-chlorobenzoyl chloride (a product of Aldrich Chemical Company) was added to the suspension and the mixture was kept at 100°C for 6 h. The reaction mixture was added to ethanol and the resulting precipitate was filtered, repeatedly washed with ethanol and vacuum-dried to obtain 9.1 g of a product. In the IR spectrum of the product, an absorption due to the ester bond (1740 $cm^{-1}$ and 1250 $cm^{-1}$) were remarkable but no absorption due to the O—H stretching vibration was recognized. This fact suggested that the product was a trisubstituted compound.

Synthesis Example 13

Synthesis of cellulose tris-3,5-dichlorobenzoate

49.4 of thionyl chloride and 0.21 g of pyridine were added to 20 g of 3,5-dichlorobenzoic acid and the mixture was refluxed for 20 h. Excessive thionyl chloride was removed by distillation. Dry hexane was added to the residue and the insoluble matter was filtered out of the resulting solution and hexane was removed under reduced pressure. The remaining solution was crystallized. 3,5-Dichlorobenzoyl chloride was obtained quantitatively.

1.0 g of the cellulose obtained in Synthesis Example 10 was reacted with 11.6 g of 3,5-dichlorobenzoyl chloride in a mixture of 25 ml of pyridine, 4.3 ml of triethylamine and 50 mg of 4-dimethylaminopyridine at 100°C for 5 h. The reaction mixture was added to ethanol and the precipitated cellulose tris-3,5-dichloro-benzoate was filtered, washed with ethanol and vacuum dried. In the IR spectrum of the product, an absorption characteristic to the ester group was recognized but no absorption at around 3500 $cm^{-1}$ due to free hydroxy group was recognized. It was thus concluded that the product was a trisubstituted compound.

Synthesis Example 14

Synthesis of cellulose tris-4-chlorobenzoate

2.43 g of the cellulose obtained in Synthesis Example 10 was reacted with 15.75 g of 4-chlorobenzoyl chloride in a mixture of 50 ml of pyridine, 20 ml of triethylamine and 200 mg of 4-dimethylaminophridine under stirring at 110°C for 8 h. The product was added to 500 ml of methanol and the resulting precipitate was filtered, washed with water and then methanol and dissolved in benzene. The resulting solution was added to ethanol to purify the product by reprecipitation. The product was filtered and vacuum-dried. In the I.R. spectrum of the product, an absorption characteristic to the ester linkage was recognized but no absorption due to free hydroxyl group at around 3500 $cm^{-1}$ was recognized. It was thus considered that the product was a trisubstituted compound.

Example 6

1.2 g of cellulose tris-(3-chlorobenzoate) obtained in Synthesis Example 12 was dissolved in 7.5 ml of dichloromethane. The silica beads obtained in Synthesis Example 9 were impregnated with 7.5 ml of the resulting solution. The solvent was removed under reduced pressure to obtain a powdery, supported material.

Example 7

1.2 g of cellulose tris-(3,5-dichlorobenzoate) obtained in Synthesis Example 13 was dissolved in 7.5 ml of dichloromethane, 3.2 g of silica beads obtained in Synthesis Example 9 were impregnated with 7.5 ml of the resulting solution. The solvent was removed under reduced pressure to obtain a powdery, supported material.

Example 8

Cellulose tris-(4-chlorobenzoate) obtained in Synthesis Example 14 was supported on silica beads in the same manner as in Example 7 to obtain a powdery material.

Application Example 2

The silica beads carrying a cellulose tris-(3-chlorobenzoate) obtained in Example 6 were packed in a stainless steel column having a length of 25 cm and an inner diameter of 0.46 cm by a slurry process. The high-performance liquid chromatograph used was TRIROTAR-SR (a product of Nihon Bunko Kogyo Co., Ltd.) and the detector was UVIDEC-V. Various racemic compounds were resolved to obtasined the results shown in Table 7.

TABLE 7

| Racemic compound | Volume ratio | | Resolution factor (α) | Rate of separation (Rs) | Flow rate (ml/min) |
|---|---|---|---|---|---|
| | $k_1'$ | $k_2'$ | | | |
| 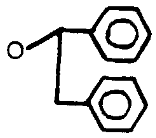 | 1.48 | 1.96 | 1.33 | 1.24 | 0.5 |
| 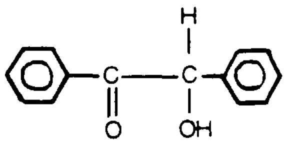 | 5.97 | 7.50 | 1.26 | 1.05 | 0.5 |
| 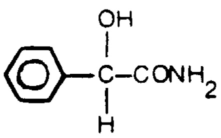 | 4.8 | 5.14 | 1.07 | — | 0.5 |
| 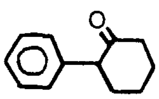 | 10.9 | 12.0 | 1.10 | — | 0.5 |

Solvent: hexane/2-propanol (9:1)

Application Example 3

The silica beads carrying cellulose tris(3,5-dichlorobenzoate) obtained in Example 7 were packed in a stainless steel column having a length of 25 cm and an inner diameter of 0.46 cm by a slurry process. The high-performance liquid chromatograph used was TRIROTAR-SR (a product of Nihon Bunko Kogyo Co., Ltd.) and the detector was UVIDEC-V. Various racemic compounds were resolved to obtain the results shown in Table 8.

TABLE 8

| Racemic compound | Volume ratio $k_1'$ | Volume ratio $k_2'$ | Resolution factor (α) | Rate of separation (Rs) | Flow rate (ml/min) |
|---|---|---|---|---|---|
| 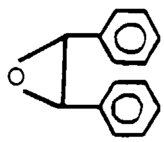 | 1.91 | 2.31 | 1.21 | 1.22 | 0.5 |
| 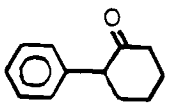 | 5.36 | 7.16 | 1.33 | 1.71 | 0.5 |
| 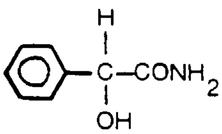 | 2.84 | 3.96 | 1.39 | 1.33 | 0.5 |
| 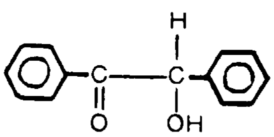 | 5.99 | 7.37 | 1.23 | 1.54 | 0.5 |

Solvent: hexane/2-propanol (9:1)

Application Example 4

The silica beads carrying cellulose tris(4-chlorobenzoate) obtained in Example 8 were packed in a stainless steel column having a length of 25 cm and an inner diameter of 0.46 cm by a slurry process. The high-performance liquid chromatograph used was TRIROTAR-SR (a product of Nihon Bunko Kogyo Co., Ltd.) and the detector was UVIDEC-V. Various racemic compounds were resolved to obtain the results shown in Table 9.

TABLE 9

| Racemic compound | Volume ratio $k_1'$ | Volume ratio $k_2'$ | Resolution factor (α) | Rate of separation (Rs) | Flow rate (ml/min) |
|---|---|---|---|---|---|
| 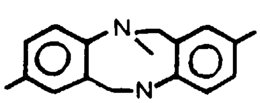 | 1.69 | 2.11 | 1.25 | 0.6 | 0.5 |
| 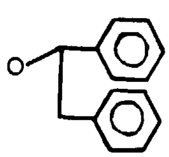 | 1.61 | 2.03 | 1.27 | 0.6 | 0.5 |

Solvent: hexane/2-propanol (9:1)

Synthesis Example 15

70 ml of dry pyridine, 7.7 ml of dry triethylamine and 50 mg of 4-dimethylaminopyridine were added to 1.5 g of the cellulose obtained in Synthesis Example 10. 12.9 g of phenylacetyl chloride was added to the mixture under stirring and the reaction was carried out at 100°C for 5 h. After cooling, the product was added to 400 ml of ethanol under stirring to form a precipitate, which was filtered through a glass filter and washed thoroughly with ethanol. After drying in vacuum, the product was dissolved in 30 ml of methylene chloride to remove the insoluble matter and reprecipitated with 400 ml of ethanol. The precipitate was filtered and washed with ethanol. After dehydration followed by drying, 4.3 g of cellulose phenylacetate was obtained.

The product was dissolved in methylene chloride and the solution was applied on a plate of common salt and dried. The I.R. absorption spectrum of the product had the following characteristic absorption bands:

3050 $cm^{-1}$ : C—H stretching vibration of the aromatic ring
1750 $cm^{-1}$ : C=O stretching vibration of the carboxylic ester group
1610 $cm^{-1}$, 1500 $cm^{-1}$, 1460 $cm^{-1}$: skeletal vibration due to C—C stretching of the benzene ring carbon atoms
1250 $cm^{-1}$: C—O stretching vibration of the ester group
1030~1160 $cm^{-1}$: C—O—C stretching vibration of cellulose
690~900 $cm^{-1}$ : out-of-plane deformation vibration of the benzene ring.

Substantially no absorption due to OH of the cellulose at around 3450 $cm^{-1}$ was recognized. The product was substantially a trisubstituted compound. Its proton NMR spectrum in $CDCl_3$ had the following characteristic resonances:

6.0 to 7.8 ppm: proton of the benzene ring
3 to 4 ppm: methylene proton of the phenylacetyl acid group
3 to 5.4 ppm: protons of the cellulose ring and methylene on position 6.

Example 9

1.2 g of the product obtained in Synthesis Example 15 was dissolved in 7.5 ml of dichloromethane. The silica gel beads obtained in Synthesis Example 9 were impregnated with 7.5 ml of the resulting solution. The solvent was distilled off under reduced pressure to obtain a powdery, supported material.

Application Example 5

The silica beads carrying cellulose trisphenylacetate obtained in Example 9 were packed in a stainless steel column having a length of 25 cm and an inner diameter of 0.46 cm by a slurry process. The high-performance liquid chromatograph used was TRIROTAR-RS (a product of Nihon Bunko Kogyo Co., Ltd.) and the detector was UVIDEC-V.

Troger's base was resolved to obtain the results shown in Table 10.

TABLE 10 Optical resolution of various racemic compounds

| Racemic compound | Volume ratio $k_1'$ | $k_2'$ | Resolution factor α | Flow rate ml/min |
|---|---|---|---|---|
| 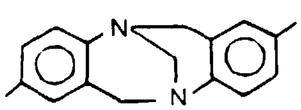 | 1.46 | 1.59 | 1.09 | 0.5 |

Solvent: hexane/2-propanol (9:1)

## Claims

1. A method of separating optical isomers and geometrical isomers from a mixture by treating said mixture with a cellulose derivative having a number-average degree of polymerization of from 5 to 5000 supported on a carrier wherein at least a part of the hydrogen atoms in the hydroxy groups has been replaced by an aromatic group or

a group containing an aromatic group, wherein said group may be attached to said cellulose derivative by means of an intermediate ester, ether or urethane linkage.

2. A method of separating optical isomers and geometrical isomers from a mixture by treating said mixture with a cellulose derivative having a number-average degree of polymerization of from 5 to 5000 wherein at least a part of the hydrogen atoms in the hydroxy groups has been replaced by a substituted aromatic group or a group containing a substituted aromatic group, wherein said group may be attached to said cellulose derivative by means of an intermediate ester, ether or urethane linkage.

3. A method of separating optical isomers and geometrical isomers from a mixture by treating said mixture with a cellulose derivative having a number-average degree of polymerization of from 5 to 5000 shaped into beads wherein at least a part of the hydrogen atoms in the hydroxy groups has been replaced by an aromatic group or a group containing an aromatic group, wherein said group may be attached to said cellulose derivative by means of an intermediate ester, ether or urethane linkage.

4. The method of any of claims 1 to 3, wherein the group containing an aromatic ring is an aromatic group having 6 to 20 carbon atoms or an aralkyl group having 6 to 20 carbon atoms in the aryl portion and 1 to 4 carbon atoms in the alkyl portion.

5. The method of any of claims 1 or 3 wherein the group containing an aromatic ring has been substituted by a substituent.

6. The method of any of claims 1 to 3 wherein the cellulose derivative is provided in a chromatographic column or as a layer.

**Patentansprüche**

1. Verfahren zum Trennen optischer Isomere und geometrischer Isomere aus einer Mischung durch Behandeln der Mischung mit einem Cellulosederivat mit einem anzahlgemittelten Polymerisationsgrad von 5 bis 5000, das auf einen Träger aufgebracht ist, wobei mindestens ein Teil der Wasserstoffatome in den Hydroxylgruppen durch eine aromatische Gruppe oder eine Gruppe, die eine aromatische Gruppe enthält, ersetzt wurde, wobei die Gruppe an das Cellulosederivat mittels einer dazwischenliegenden Ester-, Ether- oder Urethanbindung gebunden sein kann.

2. Verfahren zum Trennen optischer Isomere und geometrischer Isomere aus einer Mischung durch Behandeln der Mischung mit einem Cellulosederivat mit einem anzahlgemittelten Polymerisationsgrad von 5 bis 5000, wobei mindestens ein Teil der Wasserstoffatome in den Hydroxylgruppen durch eine substituierte aromatische Gruppe oder eine Gruppe, die eine substituierte aromatische Gruppe enthält, ersetzt wurde, wobei die Gruppe an das Cellulosederivat mittels einer dazwischenliegenden Ester-, Ether- oder Urethanbindung gebunden sein kann.

3. Verfahren zum Trennen optischer Isomere und geometrischer Isomere aus einer Mischung durch Behandeln der Mischung mit einem Cellulosederivat mit einem anzahlgemittelten Polymerisationsgrad von 5 bis 5000, das in Form von Perlen vorliegt, wobei mindestens ein Teil der Wasserstoffatome in den Hydroxylgruppen durch eine aromatische Gruppe oder eine Gruppe, die eine aromatische Gruppe enthält, ersetzt wurde, wobei die Gruppe an das Cellulosederivat mittels einer dazwischenliegenden Ester-, Ether- oder Urethanbindung gebunden sein kann.

4. Verfahren nach einem der Ansprüche 1 bis 3, wobei die Gruppe, die einen aromatischen Ring enthält, eine aromatische Gruppe mit 6 bis 20 Kohlenstoffatomen oder eine Aralkylgruppe mit 6 bis 20 Kohlenstoffatomen im Arylteil und 1 bis 4 Kohlenstoffatomen im Alkylteil ist.

5. Verfahren nach einem der Ansprüche 1 oder 3, wobei die Gruppe, die einen aromatischen Ring enthält, durch einen Substituenten substituiert worden ist.

6. Verfahren nach einem der Ansprüche 1 bis 3, wobei das Cellulosederivat in einer chromatografischen Säule oder als eine Schicht vorgesehen ist.

**Revendications**

1. Procédé de séparation d'isomères optiques et d'isomères géométriques d'un mélange par traitement dudit mélange avec un dérivé de cellulose ayant un degré de polymérisation moyen en nombre de 5 à 5000, supporté sur un véhicule, dans lequel au moins une partie des atomes d'hydrogène des groupes hydroxyle a été remplacée par un groupe aromatique ou un groupe contenant un groupe aromatique, ledit groupe pouvant être fixé audit dérivé de cellulose par une liaison intermédiaire de type ester, éther ou uréthane.

2. Procédé de séparation d'isomères optiques et d'isomères géométriques d'un mélange par traitement dudit mélange avec un dérivé de cellulose ayant un degré de polymérisation moyen en nombre de 5 à 5000, dans lequel au moins une partie des atomes d'hydrogène des groupes hydroxyle a été remplacée par un groupe aromatique substitué ou un groupe contenant un groupe aromatique substitué, dans lequel ledit groupe peut être fixé au dérivé de cellulose par une liaison intermédiaire de type ester, éther ou uréthane.

3. Procédé de séparation d'isomères optiques et d'isomères géométriques d'un mélange, par traitement dudit mélange avec un dérivé de cellulose ayant un degré de polymérisation moyen en nombre de 5 à 5000, sous la forme de perles, dans lequel au moins une partie des atomes d'hydrogène des groupes hydroxyle a été remplacée par un groupe aromatique ou un groupe contenant un groupe aromatique, ledit groupe pouvant être fixé au dérivé de cellulose par une liaison intermédiaire de type ester, éther ou uréthane.

4. Procédé selon l'une quelconque des revendications 1 à 3, dans lequel le groupe contenant un noyau aromatique est un groupe aromatique présentant de 6 à 20 atomes de carbone ou un groupe aralkyle présentant de 6 à 20 atomes de carbone dans la partie aryle et de 1 à 4 atomes de carbone dans la partie alkyle.

5. Procédé selon l'une quelconque des revendications 1 ou 3, dans lequel le groupe contenant un noyau aromatique est substitué par un substituant.

6. Procédé selon l'une quelconque des revendications 1 à 3, dans lequel le dérivé de cellulose est disposé dans une colonne chromatographique ou sous la forme d'une couche.

Fig. 1

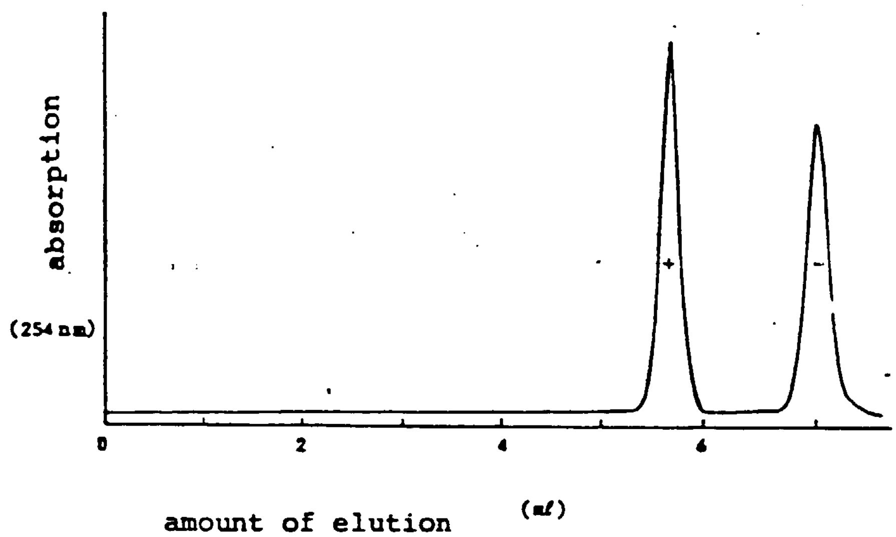
absorption
(254 nm)
+
-
0
2
4
6
amount of elution
(ml)

Fig. 2

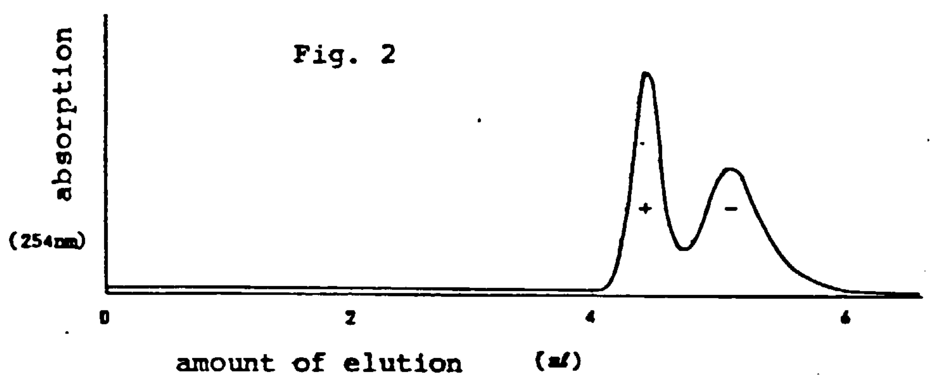
absorption
(254nm)
+
-
0
2
4
6
amount of elution
(ml)